(19) Europäisches Patentamt
European Patent Office
Office européen des brevets

(11) Numéro de publication : **0 038 246**
**B1**

(12) # FASCICULE DE BREVET EUROPÉEN

(45) Date de publication du fascicule du brevet : **15.12.82**

(51) Int. Cl.³ : **C 07 C175/00**, A 61 K 31/20, A 61 K 7/48

(21) Numéro de dépôt : **81400530.2**

(22) Date de dépôt : **02.04.81**

(54) **Le rétinoate de zinc, sa préparation et les compositions topiques le contenant.**

(30) Priorité : **04.04.80 FR 8007720**

(43) Date de publication de la demande : **21.10.81 (Bulletin 81/42)**

(45) Mention de la délivrance du brevet : **15.12.82 Bulletin 82/50**

(84) Etats contractants désignés : **AT BE CH DE FR GB IT LI LU NL SE**

(56) Documents cités :
**FR A 2 081 478**
**FR A 2 128 148**
**GB A 1 466 062**

(73) Titulaire : **PIERRE FABRE S.A.**
**125, rue de la Faisanderie**
**F-75116 Paris (FR)**

(72) Inventeur : **Navarro, Roger**
**Chemin des Farguettes Lambert**
**F-81100 Castres (FR)**
Inventeur : **Mouzin, Gilbert, Dr. en Chim.**
**21, rue Sainte-Foy**
**F-81100 Castres (FR)**
Inventeur : **Cousse, Henri, Dr. en Chim.**
**La Foun de los Noblos Chemin de Lastinos**
**F-81100 Castres (FR)**

(74) Mandataire : **Corre, Jacques Denis Paul et al**
**Cabinet Regimbeau 26, Avenue Kléber**
**F-75116 Paris (FR)**

**EP 0 038 246 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Le rétinoate de zinc, sa préparation et les compositions topiques le contenant

La présente invention, réalisée au Centre de Recherches PIERRE FABRE, concerne le rétinoate de zinc de formule

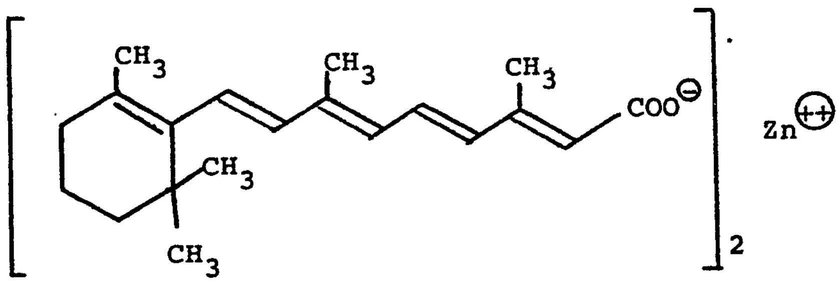

Ce nouveau dérivé de l'acide rétinoïque possède des propriétés physicochimiques et biologiques permettant son utilisation dans des préparations topiques dermatologiques et/ou cosmétologiques, et notamment pour retarder la sénescence cutanée.

Ce nouveau produit s'est en outre avéré efficace dans le traitement des divers troubles de la peau et du cuir chevelu, parmi lesquels on peut citer les dermites séborrhéiques, l'acné, les parakératoses, la desquamation grasse du cuir chevelu (pellicules) et les alopécies. En outre il possède des propriétés de cicatrisation.

Selon la présente invention, l'acide rétinoïque est associé au zinc sous la forme d'un sel ; ces deux constituants associent leurs effets complémentaires favorables pour l'application dermatologique et/ou cosmétologique, à savoir pouvoir anti-oxydant, effet kératolytique, régénération cellulaire, régulation de l'hyperfonctionnement des glandes sébacées.

Il est connu que la vitamine A acide (acide rétinoïque) est relativement instable. En outre, dès 1968 CHIKATORO KAWASAKI et coll. avaient constaté une rapide dégradation de l'acide rétinoïque en présence d'ions métalliques qui vraisemblablement catalysent l'oxydation de cet acide (référence Bitamin *37*, 204-8, 1968).

La présente invention concerne également la préparation de rétinoate de zinc. Conformément à la présente invention, le rétinoate de zinc peut être obtenu notamment sous forme de complexes d'inclusion avec le polyéthylène glycol, la polyvinyl pyrrolidone, le polysorbate 80 ou l'urée.

Le nouveau composé, objet de la présente invention, peut être obtenu par exemple de la manière suivante :

1re variante : Transalification

A 1 mole d'acide rétinoïque en suspension dans l'eau, on ajoute une solution de soude N jusqu'à neutralisation. Par addition de 0,5 mole de sulfate de zinc on provoque la formation de rétinoate de zinc qui précipite et peut être récupéré par filtration.

2e variante

Le complexe d'inclusion avec le polyéthylène glycol 400 peut être obtenu directement en traitant une solution de rétinoate de sodium dans le mélange eau-polyéthylène glycol 400 (50/50) par une quantité stœchiométrique de chlorure ou de sulfate de zinc. Le rétinoate de zinc formé se trouve enrobé par le polyéthylène glycol ; la présence d'eau empêche la formation de co-précipité et permet d'obtenir une suspension stable, homogène qui peut être utilisée directement dans les préparations dermatologiques et/ou cosmétologiques.

Caractéristiques du produit obtenu selon la première variante

Le rétinoate de zinc obtenu selon la première variante avec un rendement de 85 à 100 % possède les caractéristiques suivantes :

Formule brute : $C_{40}H_{54}O_4Zn$

Masse moléculaire : 664,24

Teneur en zinc : 10 %

Point de fusion instantané : 140 °C

Spectre IR : principaux pics d'absorption à 2 950 $cm^{-1}$, 1 690 $cm^{-1}$ ; 1 610 $cm^{-1}$ ; 1 575 $cm^{-1}$ ; 1 420 $cm^{-1}$ ; 1 260 $cm^{-1}$ ; 1 190 $cm^{-1}$ et 970 $cm^{-1}$.

Solubilités : très soluble dans l'éther éthylique, le chloroforme et l'acétone.

Caractéristiques du produit obtenu selon la deuxième variante (avec $ZnSO_4$)

Composition du produit :

$1,5 \cdot 10^{-3}$ mole de rétinoate de zinc
$1,5 \cdot 10^{-3}$ mole de sulfate de sodium
50 g eau
50 g polyéthylène glycol 400
Teneur en zinc : 0,1 %
Densité : $d_4^{20} = 1,064$
Indice de réfraction : $n_D^{20} = 1,399$
pH = 7.

Le rétinoate de zinc notamment sous forme de complexe d'inclusion, a été étudié, du point de vue de sa tolérance locale, comparativement à l'acide rétinoïque.

Pour chaque composé la tolérance a été recherchée sur cinq cobayes albinos adultes.

L'application de produit 3 fois par semaine pendant deux semaines sur le flanc préalablement rasé montre que la tolérance cutanée du rétinoate de zinc est nettement meilleure qu'avec l'acide rétinoïque.

Applications :

Compte tenu des effets complémentaires de l'acide rétinoïque associé au zinc sous forme de sel (pouvoir anti-oxydant, effet kératolytique, régénération cellulaire et régulation de l'hyperfonctionnement des glandes sébacées), le rétinoate de zinc peut être utilisé en cosmétologie et en dermatologie et plus particulièrement dans le traitement de la sénescence cutanée et de divers autres troubles cutanés.

Le rétinoate de zinc selon la présente invention peut être appliqué localement sur la peau et/ou sur le cuir chevelu, sous forme liquide ou pâteuse.

A titre d'exemple non limitatif, on indiquera ci-après quelques formulations dans lesquelles le composé de l'invention peut être utilisé seul ou associé à d'autres composants doués de propriétés cosmétologiques dans des excipients appropriés.

Formule 1 : Suspension à 0,1 % (anti-acnéique et anti-séborrhéique)

— rétinoate de zinc 0,1 g pour 100 ml
— conservateur
— polyéthylène glycol
— eau

Formule 2 : Crème dermique (anti-acnéique, anti-séborrhéique, cicatrisant, anti-sénescence cutanée)

— rétinoate de zinc 50 à 500 mg pour 100 g
— stéarate de polyéthylène glycol
— alcool stéarylique
— acide stéarique
— conservateur
— eau

Formule 3 : Gel dermique

— rétinoate de zinc 25 mg pour 100 g
— alcool éthylique
— Klucel H.F.
— conservateur

Formule 4 : Composition pour traitement anti-pelliculaire

— rétinoate de zinc 0,1 à 1 %
— polyéthylène glycol
— alcool éthylique

Formule 5 : Shampooing anti-pelliculaire et contre la chute des cheveux

— rétinoate de zinc 0,1 à 1 %
— alkyl éther sulfate de sodium 10 %
— dihydroxyéthanolamide d'acide gras 5 %
— colorant — parfum conservateur q.s.
— eau

**Revendications**

1. Rétinoate de zinc répondant à la formule

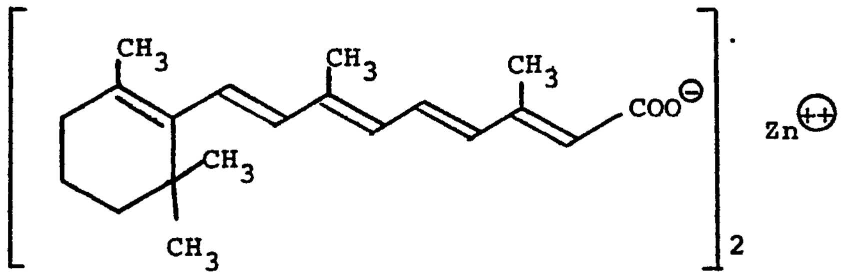

2. Le rétinoate de zinc selon la revendication 1, caractérisé en ce qu'il se présente sous la forme d'un complexe d'inclusion avec le polyéthylène glycol, la polyvinyl pyrrolidone, le polysorbate 80 ou l'urée.

3. Procédé de préparation du rétinoate de zinc, caractérisé en ce que l'on traite un sel de l'acide rétinoïque par une quantité stœchiométrique d'un sel de zinc dans un solvant.

4. Procédé selon la revendication 3, caractérisé en ce que le sel d'acide rétinoïque est le sel de sodium.

5. Procédé selon l'une des revendications 3 et 4, caractérisé en ce que le sel de zinc est choisi parmi le chlorure de zinc et le sulfate de zinc.

6. Procédé selon l'une des revendications 3 à 5, caractérisé en ce que le solvant réactionnel est choisi parmi l'eau, le polyéthylène glycol, la polyvinyl pyrrolidone, le polysorbate 80, l'urée et leurs mélanges.

7. Application du rétinoate de zinc en tant que substance active de compositions topiques dermatologiques et/ou cosmétologiques.

8. Les compositions topiques dermatologiques et/ou cosmétologiques contenant, en tant que principe actif, le rétinoate de zinc seul ou associé à d'autres principes actifs au sein d'excipients compatibles avec la forme d'application.

9. Les compositions selon la revendication 8, caractérisées en ce qu'elles se présentent sous la forme de solutions, gels et crèmes dermiques destinés au traitement de diverses affections cutanées.

10. Les compositions selon la revendication 8, caractérisées en ce qu'elles se présentent sous la forme de lotions ou de shampooings destinés au traitement de diverses affections du cuir chevelu.

**Claims**

1. Zinc retinoate corresponding to the formula

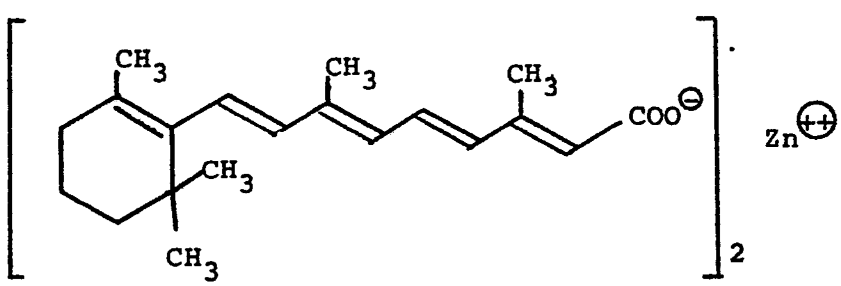

2. Zinc retinoate according to claim 1, characterised in that it is in the form of an inclusion complex with polyethylene glycol, polyvinyl pyrrolidone, polysorbate 80 or urea.

3. A process for the preparation of zinc retinoate, characterised in that a salt of retinoic acid is treated with a stoichiometric quantity of a zinc salt in a solvent.

4. A process according to claim 3, characterised in that the salt of retinoic acid is the sodium salt.

5. A process according to one of claims 3 and 4, characterised in that the zinc salt is selected from zinc chloride and zinc sulphate.

6. A process according to one of claims 3 to 5, characterised in that the reaction solvent is selected from water, polyethylene glycol, polyvinyl pyrrolidone, polysorbate 80, urea and mixtures thereof.

7. The use of zinc retinoate as an active substance in dermatological and/or cosmetic topical compositions.

8. Dermatological and/or cosmetic topical compositions containing, as the active principle, zinc

retinoate on its own or associated with other active principles in excipients which are compatible with the form of application.

9. Compositions according to claim 8, characterised in that they are in the form of dermic creams, gels and solutions for the treatment of various skin disorders.

10. Compositions according to claim 8, characterised in that they are in the form of lotions or shampoos for the treatment of various scalp disorders.

**Ansprüche**

1. Zinkretinoat entsprechend der Formel

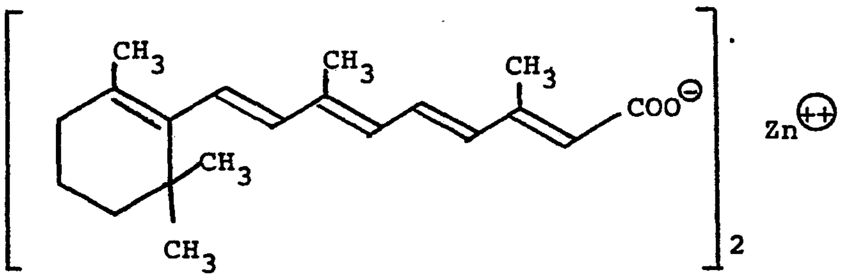

2. Das Zinkretinoat nach Anspruch 1, dadurch gekennzeichnet, daß es in Form eines Einschlußkomplexes mit Polyäthylenglycol, Polyvinylpyrrolidon, Polysorbat 80 od. Harnstoff eingesetzt wird.

3. Verfahren zur Herstellung des Zinkretinoats, dadurch gekennzeichnet, daß man ein Salz der Retininsäure mit einer stöchiometrischen Menge von Zinksalz in einem Lösungsmittel behandelt.

4. Verfahren nach Anspruch 3, dadurch gekennzeichnet, daß das Retinoat das Natriumsalz ist.

5. Verfahren nach einem der Ansprüche 3 und 4, dadurch gekennzeichnet, daß das Zinksalz unter dem Zinkchlorid und dem Zinksulfat gewählt wird.

6. Verfahren nach einem der Ansprüche 3 bis 5, dadurch gekennzeichnet, daß das Lösungsmittel unter Wasser, Polyäthylenglycol, Polyvinylpyrrolidon, Polysorbat 80, Harnstoff und deren Mischungen ausgewählt wird.

7. Anwendung des Zinkretinoats als aktive Substanz der topisch anwendbaren dermatologischen und/oder kosmetischen Zusammensetzungen.

8. Die topisch anwendbaren dermatologischen und/oder kosmetischen Zusammensetzungen, die als aktives Prinzip das Zinkretinoat allein oder in Verbindung mit anderen aktiven Prinzipien der mit der Anwendungsform verträgliche Lösungsmittel enthalten.

9. Die Zusammensetzungen nach Anspruch 8, dadurch gekennzeichnet, daß sie in Form von Lotionen, Gelees und Cremes zur Behandlung verschiedener Hautleiden eingesetzt werden.

10. Die Zusammensetzungen nach Anspruch 8, dadurch gekennzeichnet, daß sie in Form von Lotionen oder Shampoos zur Behandlung verschiedener Erkrankungen der Kopfhaut eingesetzt werden.